(19) Europäisches Patentamt
European Patent Office
Office européen des brevets

(11) Publication number: 0 004 059
B1

# (12) EUROPEAN PATENT SPECIFICATION

(45) Date of publication of patent specification: 17.02.82

(21) Application number: 79100661.2

(22) Date of filing: 06.03.79

(51) Int. Cl.³: C 07 D 233/38, A 61 K 31/415

## (54) Synthesis of 1-(thiocarbamoyl)-2-imidazolidinone and immunosuppressive compositions containing it.

(30) Priority: 06.03.78 US 884074

(43) Date of publication of application:
19.09.79 Bulletin 79/19

(45) Publication of the grant of the European patent:
17.02.82 Bulletin 82/7

(84) Designated Contracting States:
BE CH DE FR GB IT LU NL SE

(56) References cited:
GB - A - 1 230 473
GB - A - 1 255 751

HELVETICA CHIMICA ACTA, vol. 49, (fascicule 8), 10th December 1966,
BASEL (CH)
M. WILHELM et al., "Synthese und Abbau von 1-(5-nitro-2-thiazolyl)-2-imidazolidinon und Derivation", pages 2443-2452.
*Pages 2446 and 2451*

(73) Proprietor: Case Western Reserve University
Cleveland Ohio 44106 (US)

(73) Proprietor: NORTHWESTERN UNIVERSITY
Evanston
Illinois60201 (US)

(72) Inventor: Webster, Leslie T. c/o School of Medicine
Dept. of Pharmacology Case Western Reserve Univ.
Cleveland, Ohio 44106 (US)
Inventor: Warren, Kenneth S.
Rockefeller Foundation 1133 Avenue of the Americas
New York New York 10036 (US)

(74) Representative: Werner, Hans-Karsten, Dr. et al,
Deichmannhaus am Hauptbahnhof
D-5000 Köln 1 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 004 059 B1

Synthesis of 1-(thiocarbamoyl)-2-imidazolidinone, and immunosuppressive compositions containing it

The present invention relates to the production of a new composition of matter of high immunosuppressive activity.

The compound 1-(thiocarbamoyl)-2-imidazolidinone of the formula

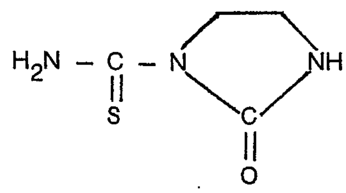

synthesized in Helv. Chem. Acta Vol. 49 (1966) pages 2443—6 is disclosed as an intermediate in making other, structurally remote compounds of niridazole-like activity against schistosoma, parasites which enter the blood streams and debilitate millions of people in Asia and Africa. Niridazole, i.e./-(5-nitro-2-thiazolyl)-2-imidazolidinone, is the treatment of choice.

It has now been found that this 1-(thiocarbamoyl)-2-imidazolinone intermediate is markedly active in suppressing immune responses such as might be encountered in tissue grafts, and the like.

The product can be prepared chemically according to the following reaction scheme:

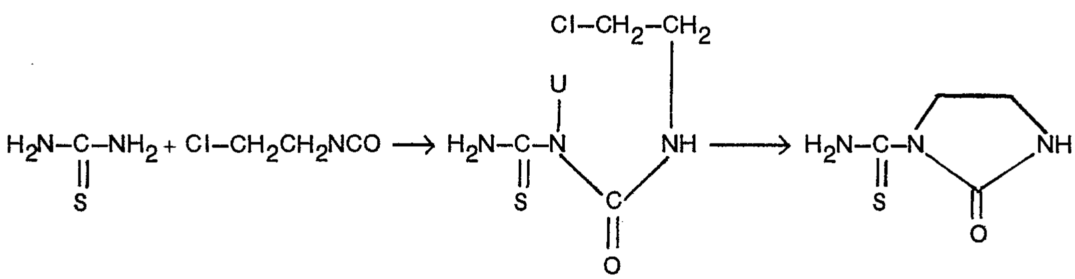

The starting materials react in a one-to-one stoichiometry but advantageously thiourea is employed in excess, e.g. at least about six times the stoichiometric amount, to minimize substitution at the second free thioamide nitrogen. The reactions can be effected in water, water-miscible solvents such as lower alkanols, acetone or dioxane, or water-immiscible solvents such as benzene or other hydrocarbons, chlorobenzene and the like. The reaction temperature can be up to the boiling point of the solvent, if present, preferably at about 20 to 70°C. Ring closure can be effected with the aid of an alkaline acid-binding agent, e.g. alkali metal or ammonium hydroxide or carbonates or tertiary amines such as pyridine, trimethylamine, triethylamine, and the like. The by-product salt which forms is watersoluble and in that manner can be separated from the desired product which can then be recrystallized from ethanol.

An illustrative process for the preparation of the active material synthetically is shown in the following example:

EXAMPLE 1

To a solution of 19.0 g (250 nmol) of thiourea in 400 ml of acetone, containing 12.5 g (90 mmol) of anhydrous potassium carbonate, which was heated to 50°C, was added dropwise with stirring over a 90 minute period, a solution of 5.0 g (47.4 mmol) of 2-chloroethyl isocyanate diluted in 150 ml of acetone. The reaction mixture was stirred for two more hours at 50°C. After the insoluble carbonate was removed by filtration, the solution was evaporated to dryness at 45°C under reduced pressure. The solids which remained were dissolved in 375 ml of N,N-dimethylformamide and heated to 94°C. Fourteen grams of sodium acetate trihydrate were added in one lot and the mixture stirred for 30 minutes at 94°C. After the solution had cooled to 45°C, the solvent was removed by rotary evaporation.

The solids were dissolved in 100 ml of water. Upon standing at 4°C, 1.8 g of crystals were deposited which contained approximately 90% by weight of a product.

Recrystallization from 60 ml of water gave 1.44 g of white crystals which melted at 203.5 to 205.5°C (uncorrected) and which were substantially free of thiourea. Traces of the starting material were removed by chromatography of the product in 250 mg lots on a Sephadex® LH—20 (5 × 60 cm) which was developed with water. Under these conditions thiourea eluted after about one bed volume of solvent had passed through, whereas the major product eluted after about two bed volumes.

Finally, highly purified product (ν 99% by weight) was obtained in milligram quantities by preparative high pressure liquid chromatography, using water as the solvent, as follows:

A $\mu$Bondapak® C18 column was washed with 10 column volumes of acetonitrile (Burdick-Jackson, uv-quality solvent) and then equilibrated with water which was highly purified by passage through a Milli-Q system (Millipore Corporation). When chromatography was done in water at a flow rate of 4.0 ml/minute, the active material eluted with a retention time of 21.6 minute. Again the column effluent was monitored at 256 nm and at the appropriate time, active material was collected. The volume of the collected effluent was reduced to 4 ml by rotary evaporation at 30°C. Product was recovered as fine white powder after lyophilization.

When foreign tissues enter a human body, the body's immune system produces a "migration inhibitory factor" (MIF) to reject such tissues. Agents which block or prevent the production of MIF are termed "immunosuppressive agents" and are effective in reducing allergic and rejection responses as in organ and tissue transplants.

The active material synthesized hereinabove exhibits such activity which can be demonstrated clearly with laboratory animals, e.g. by an assay procedure such as that described by David and David at page 249 of their "In Vitro Methods in Cell-Mediated Immunity", edited by Bloom et al (Academic Press, 1971). A capillary tube technique with modified Mackaness chambers is employed to measure the MIF activity resulting from the addition of various levels of OCB—BGG and get to peritoneal exudate cells (PEC) obtained from Hartley guinea pigs sensitized to this antigen. The culture medium is Eagle's Minimal Essential Medium with penicillin (100 units/ml) and streptomycin (100 $\mu$g/ml), and is made to contain 15% normal guinea pig serum. Chambers are incubated for 24 hours at 37°C. Areas of migration are projected, traced, and quantitated by planimetry, MIF production being expressed as percent inhibition of migration, calculated as:

$$\% \text{ inhibition} = \left(1 - \frac{\text{area of migration in presence of antigen}}{\text{area of migration in presence of antigen}}\right) \times 100$$

Twenty percent or greater inhibition of migration usually represents significant MIF production by this assay according to the Student's *t* test.

More specifically, the compound of the invention, 1-(thiocarbamoyl)-2-imidazolidinone, at 100 ng./ml., significantly inhibits ($p \leq .01$) macrophage migration inhibitory factor (MMIF), which is produced by incubation of sensitized guinea pig lymphocytes in the presence of 125 units/ml. of PPD (purified protein derivative) as antigen. [The measurement of inhibition of MMIF to determine immunosuppressant activity is described by Bloom et al., Science *153*, 80—82 (1966); David et al., J. Immunol. *93*, 264—273 (1964); George et al., Proc. Soc. Exp. Biol. Med. *111*, 514—521 (1962); and Kreici et al., Immunology *16*, 677—684 (1969)].

As shown by the data presented in Table 1 below, using the Jerne-plaque assay described by Jerne et al., Cell Bound Antibody (Amos and Koprowski, eds.), pages 109—125, Wistar Institute Press, Philadelphia (1963), 1-(thiocarbamoyl)-2-imidazolidinone, identified as "Cpd.", was found to suppress IgM antibody (immunoglob lin M, 19S) at concentrations from 0.01 to 0.1 mg./kg. against sheep red blood cells as antigen when administered orally to mice for three days. For purposes of comparison, data are also included for the known immunoregulator, Tilorone, 2,7-bis-[2-(diethylamino)ethoxy]fluoren-9-one, identified as "Ref.". Results are expressed in terms of the Immunomodulation Index, which is the ratio of the PFC/$10^6$ Lymphocyte count for the test compound at a particular dose vs. control, a ratio greater than unity indicating immunoenhancement and a ratio less than unity indicating immunosuppression.

TABLE 1

| Medication | Dose mg./kg. | PFC/$10^6$ Lymphocytes (a) | Immunomodulation Index | p Value |
|---|---|---|---|---|
| GT (b) | | 246.1 ± 9% | 1.0 | – |
| Ref. | 25 | 353.1 ± 12% | 1.43 | $\leq$.05 |
| | 37.5 | 149.6 ± 22% | 0.61 | $\leq$.05 |
| | 50 | 173.8 ± 7% | 0.71 | $\leq$.05 |
| Cpd. | 0.01 | 124.1 ± 19% | 0.50 | $\leq$.01 |
| | 0.1 | 145.9 ± 10% | 0.59 | $\leq$.001 |
| | 1.0 | 151.1 ± 24% | 0.61 | |

(a) Plaque-forming cells – geometric mean.

(b) Gum tragacanth control.

As shown by the data presented in Table 2 below, using the Jerne-plaque assay described above, 1-(thiocarbamoyl)-2-imidazolidinone was found to enhance IgM antibody in Nu/Nu (i.e. athymic) mice at a dose of 0.1 mg./kg. (p.o.) administered in 11 daily doses beginning 7 days preceding antigen administration (sheep red blood cells) and continuing for 4 days thereafter. As before, for comparative purposes data are also given for Tilorone, and in addition the PFC/$10^6$ Lymphocyte count and the Immunomodulation Index calculated therefrom is given for heterozygous mice (Het.) which are the parents, having fully active thymi, of the athymic mice used to assess the test compounds.

TABLE 2

| Medication | Dose mg./kg. | PFC/$10^6$ Lymphocytes | Immunomodulation Index | p Value |
|---|---|---|---|---|
| GT | – | 5.1 ± 30% | 1.0 | – |
| Cpd. | 0.1 | 22.3 ± 30% | 4.4 | $\leq$.01 |
| | 1.0 | 5.6 ± 37% | 1.1 | |
| | 10.0 | 4.5 ± 16% | 0.9 | |
| Ref. | 5 | 185 ± 32% | 3.6 | $\leq$.01 |
| Het. | – | 330.9 ± 7% | 64.9 | $\leq$.001 |

## Claims

1. An immunosuppressive composition of matter comprising a pharmacologically acceptable diluent and an immunosuppressive effective amount of synthetically produced 1-(thiocarbamoyl)-2-imidazolidinone of the formula

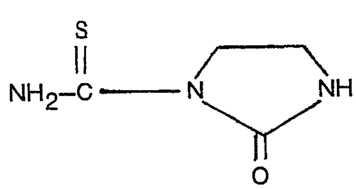

2. A process for preparing 1-(thiocarbamoyl)-2-imidazolidinone comprising reacting thiourea with $\beta$-chlorethyl-isocyanate and then with an acid binding agent.

**Revendications**

1. Composition de matière immunosuppresseur comprenant un diluant pharmacologiquement acceptable et une quantité efficace à titre d'immunosuppresseur de 1-(thiocarbamoyl)-2-imidazolidinone obtenue par synthèse, de formule:

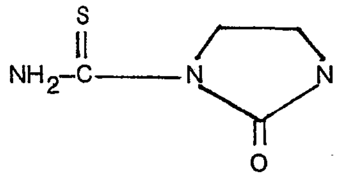

2. Procédé de préparation de 1-(thiocarbamoyl)-2-imidazolidinone consistant à faire réagir de la thiourée avec l'isocyanate de $\beta$-chloréthyle et ensuite avec un accepteur d'acide.

**Patentansprüche**

1. Immunosuppressive Stoffzusammensetzung, enthaltend ein pharmakologisch unbedenkliches Verdünnungsmittel und eine immunosuppressive wirksame Menge eines synthetisch hergestellten 1-(Thiocarbamoyl)-2-imidazolidinons der Formel.

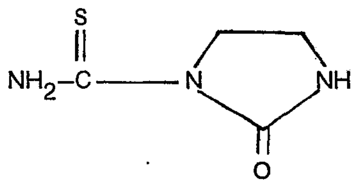

2. Verfahren zur Herstellung von 1-(Thiocarbamoyl)-2-imidazolidinon, dadurch gekennzeichnet, daß Thioharnstoff mit $\beta$-Chloroethylisocyanat und danach mit einem säurebindenden Mittel umgesetzt wird.